Europäisches Patentamt

European Patent Office

Office européen des brevets

(11) Veröffentlichungsnummer: **0 312 847**

**A1**

## (12) EUROPÄISCHE PATENTANMELDUNG

(21) Anmeldenummer: 88116588.0

(22) Anmeldetag: 06.10.88

(51) Int. Cl.4: **G10K 9/12 , A61B 17/22**

(30) Priorität: 19.10.87 DE 3735346

(43) Veröffentlichungstag der Anmeldung:
26.04.89 Patentblatt 89/17

(84) Benannte Vertragsstaaten:
DE FR GB NL

(71) Anmelder: **Siemens Aktiengesellschaft Berlin
und München**
**Wittelsbacherplatz 2**
**D-8000 München 2(DE)**

(72) Erfinder: **Hassler, Dietrich, Dipl.-Ing.**
**Flurweg 3**
**D-8525 Uttenreuth(DE)**

(54) **Stosswellenquelle mit zentralem Ultraschall-Ortungssystem.**

(57) Es wird von einem Lithotripter mit einer elektromagnetischen Stoßwellenquelle (2a, 2b, 2c) und mit
dem Ultraschallkopf (30) einer Ultraschall-Ortungseinrichtung ausgegangen. Nach der Erfindung ist der
Ultraschallkopf (30) in den zentralen Bereich der
Stoßwellenquelle (2a, 2b, 2c) einbringbar. Zusätzlich
ist bei der Ortung zumindest eine Flächennormale
(34) der Ultraschall-Austrittsfläche (32) des Ultraschallkopfs (30) schräg zur Flächennormalen (27)
der Ankoppelfläche (25) ausgerichtet. Bildstörungen
durch Mehrfachechos sind durch das Schrägstellen
der Ultraschall-Austrittsfläche (32) vermieden. Lithotripter mit Ultraschall-Ortungssystemen werden mit
Vorteil auch zur Zertrümmerung von Gallensteinen
eingesetzt.

EP 0 312 847 A1

FIG 1

## Stoßwellenquelle mit zentralem Ultraschall-Ortungssystem

Die Erfindung betrifft einen Lithotripter mit einer elektromagnetischen Stoßwellenquelle zur Aussendung akustischer Impulse mit dem Ultraschallkopf einer Ultraschall-Ortungseinrichtung. Es handelt sich also dabei um eine Einrichtung zum berührungslosen Zertrümmern eines im Körper eines Lebenwesens befindlichen Konkrements.

Eine Einrichtung der eingangs genannten Art ist in der DE-OS 33 28 068 in den Figuren 1 und 3 gezeigt. Der Ultraschallkopf einer Ultraschall-Sende- und -Empfangseinrichtung, die zum Orten und Beobachten eines Konkrements im menschlichen Körper dient, ist dort neben bzw. zwischen mehreren Stoßwellenquellen angeordnet. Als nachteilig ist bei der bekannten Einrichtung zu werten, daß die seitliche Anordnung des Ultraschallkopfes unnötiges Bauvolumen beansprucht.

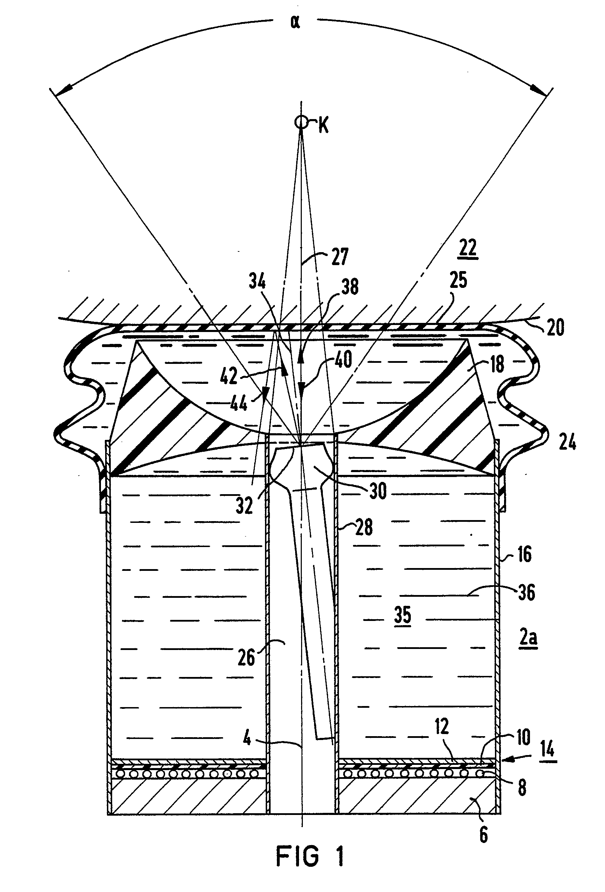

Bei einem ortsfesten Ultraschallkopf, wie dort z.B. in FIG 1 dargestellt, entstehen wegen praktisch nicht vermeidbarer Fehlanpassungen Mehrfachreflexionen zwischen dem Ultraschallkopf und der Ankoppelstelle. Wenn die empfangenen Echos dieser Mehrfachreflexionen gerade auf die Abbildungsstelle des Konkrements auf dem Monitor der Ultraschall-Ortungseinrichtung fallen, wird die Ortung und Beobachtung des Konkrements merklich gestört.

In der deutschen Patentanmeldung P 37 27 691.3 ( = VPA 87 P 3296 DE) wird vorgeschlagen, in einer Stoßwellenquelle nach dem elektromagnetischen Prinzip eine zentrale Öffnung vorzusehen, in die beim Betrieb der Ultraschallkopf eines Ultraschall-Ortungssystems eingebracht wird. Auch hier entstehen bei ortsfestem Ultraschallkopf zwischen dem Ultraschallkopf und der Ankoppelstelle Mehrfachreflexionen, deren Echos gerade an die Stelle der Konkrementabbildung fallen können.

Aufgabe der Erfindung ist es, einen Lithotripter der eingangs genannten Art so auszubilden, daß er klein und kompakt aufgebaut ist und eine exakte Beobachtung und/oder Lokalisation des Konkrements bei ortsfestem Ultraschallkopf ermöglicht.

Die genannte Aufgabe wird erfindungsgemäß dadurch gelöst, daß der Ultraschallkopf in den zentralen Bereich der Stoßwellenquelle einbringbar ist, und daß bei der Ortung zumindest eine Flächennormale der Ultraschall-Austrittsfläche des Ultraschallkopfes schräg zur Flächennormalen der Ankoppelfläche ausgerichtet ist.

Eine vorteilhafte Ausgestaltung der Erfindung zeichnet sich dadurch aus, daß die Ultraschall-Austrittsfläche des Ultraschallkopfes eben ist. Bevorzugt ist der Ultraschallkopf ein lineares Array oder Phased Array.

Bei einer weiteren vorteilhaften Ausgestaltung ist die Ultraschall-Austrittsfläche des Ultraschallkopfes kegelförmig; der Ultraschallkopf ist hierbei bevorzugt ein Annular Array. Nach einer Weiterbildung ist der Ultraschallkopf um eine Achse schwenkbar, die in der Grundfläche des kegelförmigen Teils des Ultraschallkopfes liegt. Um diese Achse wird der Ultraschallkopf im Stoßwellenbetrieb in eine Lage geschwenkt, in der er die akustischen Impulse der Stoßwellenquelle nur gering abschattet.

Weitere Ausbildungen und Vorteile der Erfindung ergeben sich aus der nachfolgenden Beschreibung von Ausführungsbeispielen anhand von drei Figuren sowie aus den Unteransprüchen.

Es zeigen:

FIG 1 einen Längsschnitt einer Stoßwellenquelle mit einem linearen Array als Ultraschallkopf einer Ortungseinrichtung;

FIG 2 einen Längsschnitt einer Stoßwellenquelle mit einem Annular Array als Ultraschallkopf einer weiteren Ortungseinrichtung; und

FIG 3 einen Längsschnitt einer Stoßwellenquelle mit einem ebenen Annular Array als Ultraschallkopf einer weiteren Ortungseinrichtung.

In FIG 1 ist mit 2a eine Stoßwellenquelle nach dem elektromagnetischen Prinzip bezeichnet. Die Stoßwellenquelle 2a ist axialsymmetrisch zu einer Zentralachse 4 ausgebildet. Somit hat sie die äußere Form eines Zylinders. An einem Ende der Stoßwellenquelle 2a befindet sich ein Spulenträger 6 mit einer Flachspule 8. An der Flachspule 8 ist auf der dem Spulenträger 6 gegenüberliegenden Seite eine Isolierfolie 10 befestigt. Sie ist bevorzugt auf die Flachspule 8 geklebt.

Ohne jeden Abstand zur Isolierfolie 10 ist eine metallische Membran 12 angeordnet. Der enge Kontakt zwischen der Isolierfolie 10 und der Membran 12 kann in bekannter Weise durch einen Unterdruck hergestellt werden.

Der Spulenträger 6, die Flachspule 8, die Isolierfolie 10 und die Membran 12 bilden den Kern 14 der Stoßwellenquelle 2a. Der Kern 14 befindet sich in dem einen Ende eines rohrförmigen Gehäuses 16. Er bildet hier den Abschluß des Gehäuses 16. Am oder im anderen Ende des Gehäuses 16 ist eine Fokussierungsvorrichtung 18 befestigt, die die akustischen Impulse oder Stoßwellen der Stoßwellenquelle 2a auf ein Konkrement K in einem Lebewesen fokussiert. Um die Stoßwellen verlustarm an der Hautoberfläche 20 in den Körper 22 des Lebewesens einzukoppeln, ist ein Ankoppelsack 24 flüssigkeitsdicht am Ende des Gehäuses 16 außen befestigt. Zur Anpassung der Stoßwellenquelle 2a auf das in gewisser Tiefe liegende Konkrement K im Körper 22 ist der Ankoppelsack 24 an seinem Umfang als Faltenbalg ausgebildet. Seine Ankoppelfläche 25 ist eben. Die Flächennormale 27 der Ankoppelfläche 25 ist parallel zur Zentralachse 4 ausgerichtet.

Zentral in der Stoßwellenquelle 2a ist ein zylindrischer Raum 26 vorgesehen, der im wesentlichen frei ist von den akustischen Impulsen, die im Stoßwellenbetrieb im Kern 14 erzeugt werden. Der Raum 26 ist begrenzt von oder ist ausgekleidet mit einem Rohr 28, das an seinem einen Ende in der Fokussierungsvorrichtung 18 und an seinem anderen Ende im Kern 14 befestigt ist. Die Längsachse des Raumes 26 fällt mit der Zentralachse 4 zusammen. In diesem Raum 26 ist der Ultraschallkopf 30 eines Ultraschall-Ortungssystems ortsfest angeordnet und flüssigkeitsdicht eingepaßt. Es handelt sich hier um ein lineares Array oder ein Phased Array (elektronischer Sektorscanner). Die Ultraschall-Austrittsfläche 32 des Ultraschallkopfes 30 ist eben ausgebildet, sie liegt nahe der Fokussierungsvorrichtung 18. Die Flächennormale 34 der Ultraschall-Austrittsfläche 32 bildet einen bestimmten, von Null verschiedenen Winkel zur Zentralachse 4 und damit auch zur Flächennormalen 27 der Ankoppelfläche 25 oder Hauptoberfläche 20. Dadurch ist vermieden, daß die Austrittsfläche 32 zur Hautoberfläche 20 parallel liegt.

Der Innenraum 35 der Stoßwellenquelle 2a ist begrenzt von der Membran 12, dem Gehäuse 16, dem Ankoppelsack 24, der Wandung 28 und einer Teiloberfläche des Ultraschallkopfes 30. Der Innenraum 35 ist gefüllt mit einer Koppelflüssigkeit 36. Aus gleichgefäße, die die Koppelflüssigkeit 36 aufnehmen oder abgeben, wenn die Stoßwellenquelle 2a auf unterschiedlich tief im Körper 22 liegende Konkremente K angepaßt wird, sind nicht gezeigt. Ebenso sind nicht gezeigt mechanische Halte- und Einstellglieder für die Stoßwellenquelle 2a. Die akustischen Eigenschaften der Koppelflüssigkeit 36, z.B. Wasser, entsprechen weitgehend denjenigen des Lebewesens 22.

Durch die schräge Ausrichtung der Normalen 34 der Ultraschall-Austrittsfläche 32 zur Zentralachse 4 werden Mehrfachreflexionen zwischen der Austrittsfläche 32 und dem Ankoppelsack 24 sowie der Hautoberfläche 20 vermieden. Mehrfachreflexionen stören besonders , wenn die hieraus herrührenden empfangenen Echosignale in der Abbildung mit dem Ort des Konkrements K zusammenfallen, d.h. wenn der Abstand zwischen der Ultraschall-Austrittsfläche 32 und dem Konkrement K ein ganzzahliges Vielfaches des Abstandes zwischen der Ultraschall-Austrittsfläche 32 und der Hautoberfläche 20 ist.

Im folgenden wird der Weg eines reflektierten Ultraschall-Strahls beschrieben. Es wird ein Ultraschall-Sendeimpuls betrachtet, der von dem Ultraschallkopf 30 in Richtung der Zentralachse 4 ausgesendet wird. Das ist mit einer Pfeilspitze 38 verdeutlicht. Dieser Sendeimpuls wird infolge der unvermeidbaren akustischen Fehlanpassung teilweise von der Hautoberfläche 20 und/oder vom Ankoppelsack 24 zurück in Richtung der Zentralachse 4 reflektiert, was durch eine Pfeilspitze 40 verdeutlicht ist. Da der reflektierte Impuls nun unter einem von Null verschiedenen Winkel zur Flächennormalen 34 auf die Ultraschall-Austrittsfläche 32 auftrifft, wird er zumindest teilweise mit dem gleichen Winkel bezüglich der Flächennormale 34 nochmals reflektiert. Das zeigt die Pfeilspitze 42. Da jetzt der reflektierte Strahl nicht mehr senkrecht auf die Hautoberfläche 20 oder auf die Ankoppelfläche 25 des Ankoppelsacks 24 auftrifft, wird er an dieser Fläche 20, 25 ebenfalls unter der Bedingung Einfallswinkel gleich Ausfallswinkel reflektiert. Der zum zweiten Mal an der Hautoberfläche 20 oder am Ankoppelsack 24 reflektierte Ultraschallimpuls trifft jetzt nicht mehr auf die Ultraschall-Austrittsfläche 32 des Schallkopfes 30 auf; der Impuls verläuft sich in der Koppelflüssigkeit 36. Mit anderen Worten: es trifft nur eine erste Reflexion 40 des Ultraschallimpulses wieder auf der Ultraschall-Austrittsfläche 32 auf. Das Echosignal der ersten Reflexion 40 gibt auf dem Monitor die Lage der Hautoberfläche 20 an.

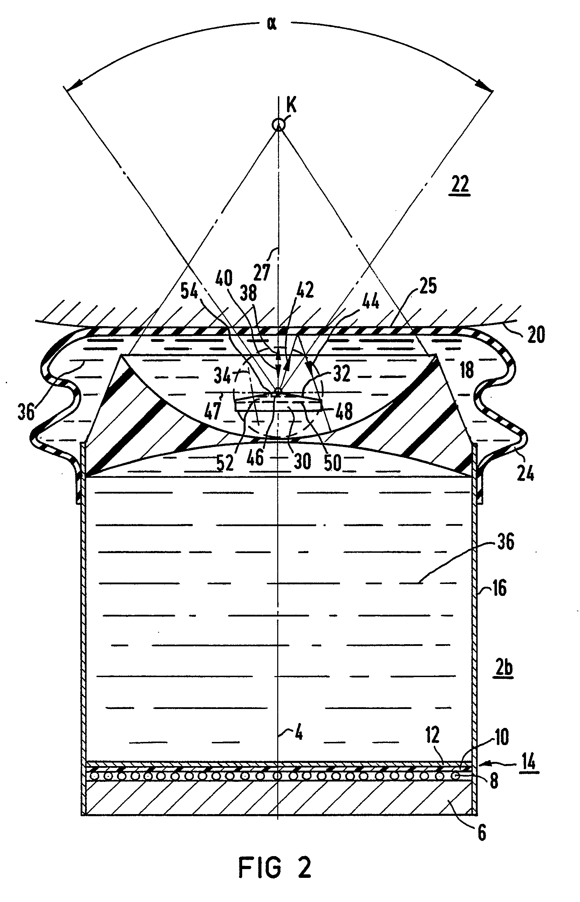

Die in FIG 2 dargestellte Stoßwellenquelle 2b ist ähnlich aufgebaut wie die Stoßwellenquelle 2a in FIG 1. Gleiche Bauteile sind mit denselben Bezugszeichen gekennzeichnet. Die Stoßwellenquelle 2b unterscheidet sich von der Stoßwellenquelle 2a in FIG 1 jedoch durch die Art und Anordnung des Ultraschall-Ortungssystems. Auch fehlt in dem in FIG 2 beschriebenen Ausführungsbeispiel der Raum 26, der frei ist von den akustischen Impulsen, die von der Stoßwellenquelle 2a erzeugt werden.

Der Ultraschallkopf 30 der Ultraschall-Ortungseinrichtung ist hier vor der äußeren Begrenzungsfläche der Fokussierungsvorrichtung 18 um eine Achse 46 drehbar gelagert. Die Achse 46 steht senkrecht auf der Zentralachse 4. Dabei ist der Ultraschallkopf 30 ein Ring- oder Annular Array, dessen Ultraschall-Austrittsfläche 32 kegelförmig ist. Die divergierende Wirkung der hier kegelförmigen Ultraschall-Austrittsfläche 32 wird durch entsprechende elektronische Ansteuerung der einzelnen Ringe kompensiert. Der Ultraschallkopf 30 oszilliert beim Orten und Beobachten des Konkrementes K um die Achse 46. Der Abtastsektor ist durch den Winkel Alpha gekennzeichnet. Es handelt sich also um einen mechanischen Sektorscanner mit elektronischer Fokussierung.

Damit die akustischen Impulse, die von der Stoßwellenquelle 2b ausgehen, nicht unnötig vom Ultraschallkopf 30 abgeschattet sind, ist der Ultraschallkopf 30 während der Aktivierung der Stoßwellenquelle 2b so weggeschwenkt, daß die Abschattung der Stoßwellen minimal ist.

Der Ultraschallkopf 30 ist manuell oder auch von einem Motor angetrieben um eine weitere Achse 47 schwenkbar. Die Achse 47 steht senkrecht auf der Fläche, in der die Zentralachse 4 und die Achse 46 liegen. Damit kann der Abtastsektor Alpha seitlich verlagert werden. Somit ist ein Raum und nicht nur eine Schnittfläche mit der Ultraschall-Ortungseinrichtung zu beobachten. Das ist von Vorteil, wenn die Lage des zu behandelnden Konkrements K noch nicht genau ermittelt ist. Dann kann, ohne die Stoßwellenquelle 2b an der Ankoppelfläche 25 zu bewegen, die Lage des Konkrements bestimmt werden. Danach wird dann die Zentralachse 4 auf das Konkrement K ausgerichtet.

Da der Ultraschallkopf 30 an der Spitze seiner kegelförmigen Ultraschall-Austrittsfläche 32 gelagert ist, ist der Durchmesser des Rotationskreises, der durch die gestrichelte Linie 48 angedeutet ist, annähernd so groß wie der Durchmesser des Ultraschallkopfs 30 selbst. Die größtmögliche Apertur des Ultraschallkopfs 30 erhält man dann, wenn die Achse 46 in der Grundfläche 50 des kegelförmigen Teils 52 des Ultraschallkopfes 30 liegt. Das Teil 52 des Ultraschallkopfes 30 ist begrenzt von der Grundfläche 50 und der kegelförmigen Ultraschall-Austrittsfläche 32. Große Aperturen erreicht man auch, wenn sich die Achse 46 in einer kreisförmigen Schnittfläche 54 befindet, die zwischen der Spitze des Teils 52 und der Grundfläche 50 liegt.

Die spezielle kegelförmige Ultraschall-Austrittsfläche 32 verhindert ebenfalls störende Mehrfachreflexionen. Auch hier ist ein ausgesendeter Ultraschall-Ortungsimpuls durch den Pfeil 38 gekennzeichnet. Dieser Impuls wird an der Hautoberfläche 20 und/oder am Ankoppelsack 24 wegen der unvermeidlichen akustischen Fehlanpassungen teilweise reflektiert. Die Richtung des reflektierten Impulses deckt sich mit der Richtung des ausgesendeten Impulses, was durch die Pfeilspitze 40 verdeutlicht ist. Dieser reflektierte Strahl tritt nun auf die kegelförmige Ultraschall-Austrittsfläche 32 auf. Da die Richtung des auftreffenden Impulses nicht mit der Flächennormalen 34 übereinstimmt, wird der Strahl, entsprechend dem Gesetz Einfallswinkel gleich Ausfallswinkel, in Richtung des Pfeiles 42 von der Ultraschall-Austrittsfläche 32 reflektiert. Nach der zweiten Reflexion an der Hautoberfläche 20 und/oder am Ankoppelsack 24, verdeutlicht durch den Pfeil 44, trifft der Ultraschall-Ortungsimpuls nicht mehr auf die aktive Fläche des Ultraschallkopfes 30 auf. Die erste Reflexion 40 gibt auf dem Monitor des Ultraschall- Ortungssystems die Lage der Hautoberfläche 20 an.

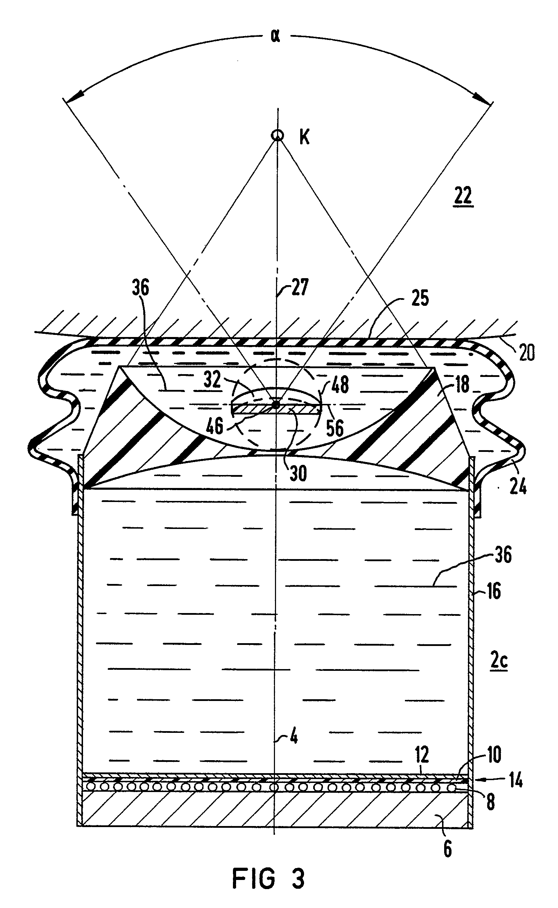

Nach einer weiteren Ausführung, die in FIG 3 gezeigt ist, ist die Ultraschall-Austrittsfläche 32 eben ausgeführt. Der Aufbau einer Stoßwellenquelle 2c ist ähnlich wie in FIG 2, für gleiche Teile werden gleiche Bezugzeichen verwendet.

Der Ultraschallkopf 30 ist hier gleichmäßig dick, d.h. zylindrisch ausgebildet. Er ist um eine Kippachse 56, die senkrecht auf der Zentralachse 4 und der Schwenkachse 46 steht, so gekippt, daß die Flächennormale 34 (hier nicht dargestellt) der Ultraschall-Austrittsfläche 32 einen von Null verschiedenen Winkel mit der Zentralachse 4 bildet. Es handelt sich hier um ein lineares Array. Seine einzelnen Teilwandler sind in Richtung der Achse 46 hintereinander angeordnet. Durch entsprechend elektronische Ansteuerung der Teilwandler ist die schräge Ausrichtung der Ultraschall-Austrittsfläche 32 kompensiert. Die Abtastung erfolgt also auch hier in einer Ebene, in der die Zentralachse liegt. Dadurch sind auch hier, wie in den in FIG 1 und 2 beschriebenen Ausführungen, Mehrfachreflexionen ausgeschlossen. Es trifft auch hier nur das Echo der ersten Reflexion eines entlang der Zentralachse 4 ausgesendeten Ultraschallimpulses wieder auf die aktive Fläche, also auf die Ultraschall-Austrittsfläche 32, auf. Darauffolgende Reflexionen an der Hautoberfläche 20 und/oder dem Ankoppelsack 24 laufen am Ultraschallkopf vorbei. Die weiteren Einzelheiten sind identisch mit der Stoßwellenquelle 2b nach FIG 2.

In dem in FIG 1 beschriebenen Ausführungsbeispiel ist der Ultraschallkopf 30 des elektronischen Sektorscanners (Phased Array) nicht bis an den Ankoppelsack 24 gebracht; er befindet sich vielmehr in einem vorgegebenen Abstand zum Ankoppelsack 24 in einer Öffnung der Fokussierungsvorrichtung 18. Damit bleibt die Abschattung des Stoßwellenimpulses gering. Bildstörungen durch Mehrfachechos sind durch das Schrägstellen der Ultraschall-Austrittsfläche vermieden.

Bei den in FIG 2 und 3 gezeigten Ausführungsbeispielen mit einem mechanischen Sektorscanner, der elektronisch fokussiert ist (Annular Array bzw. lineares Array), ist die Abschattung durch Wegschwenken des Wandlers 30 gering gehalten. Auch hier treten Störungen im Bild aufgrund von Mehrfachechos durch die kegelförmige bzw. schräg ausgerichtete Ultraschall-Austrittsfläche 32 nicht auf.

Lithotripter mit Ultraschall-Ortungssystemen werden mit Vorteil auch zur Zertrümmerung von Gallensteinen eingesetzt. Bei der zentralen Anordnung des Ultraschall-Ortungssystems wird der gleiche optimale Ultraschallzugangsweg zum Konkrement K benutzt, den die Stoßwelle passiert. Weiterhin werden Mehrfachreflexionen, die die Beobachtung und Ortung des Konkrementes K stören könnten, durch die schräge Ausrichtung der Ultraschall-Austrittsfläche 32 vermieden. Man erhält ein artefaktfreies Bild des beobachteten Gebietes.

## Ansprüche

1. Lithotripter mit einer elektromagnetischen Stoßwellenquelle zur Aussendung akustischer Impulse und mit dem Ultraschallkopf einer Ultraschall-Ortungseinrichtung, **dadurch gekennzeichnet,** daß der Ultraschallkopf (30) in den zentralen Bereichen der Stoßwellenquelle (2a, 2b, 2c) einbringbar ist, und daß bei der Ortung zumindest eine Flächennormale (34) der Ultraschall-Austrittsfläche (32) des Ultraschallkopfes (30) schräg zur Flächennormalen (27) der Ankoppelfläche (25) ausgerichtet ist.

2. Lithotripter nach Anspruch 1, **dadurch gekennzeichnet,** daß die Ultraschall-Austrittsfläche (32) des Ultraschallkopfes (30) eben ist.

3. Lithotripter nach Anspruch 2, **dadurch gekennzeichnet,** daß der Ultraschallkopf (30) ein lineares oder Phased Array ist.

4. Lithotripter nach Anspruch 1, **dadurch gekennzeichnet,** daß die Ultraschall-Austrittsfläche (32) des Ultraschallkopfes (30) kegelförmig ist.

5. Lithotripter nach einem der Ansprüche 1, 2 oder 4, **dadurch gekennzeichnet,** daß der Ultraschallkopf (30) ein Annular Array ist.

6. Lithotripter nach einem der Ansprüche 1 bis 5, **dadurch gekennzeichnet,** daß der Ultraschallkopf (30) um eine Achse (46) schwenkbar ist.

7. Lithotripter nach Anspruch 6, **dadurch gekennzeichnet,** daß die Achse (46) senkrecht auf der Zentralachse (4) der Stoßwellenquelle (2) steht.

8. Lithotripter nach Anspruch 4 und 6, **dadurch gekennzeichnet,** daß die Achse (46) in einer kreisförmigen Schnittfläche (54) zwischen der Grundfläche (50) und der Spitze des kegelförmigen Teiles (52) des Ultraschallkopfes (30) liegt.

9. Lithotripter nach Anspruch 8, **dadurch gekennzeichnet,** daß die Schnittfläche (54) mit der Grundfläche (50) zusammenfällt, und daß die Achse (46) in der Grundfläche (50) liegt.

10. Lithotripter nach einem der Ansprüche 1 bis 9, **dadurch gekennzeichnet,** daß der Ultraschallkopf (30) sich in einem Raum (26) befindet, der beim Senden der Stoßwellenquelle (2) frei von akustischen Impulsen ist.

11. Lithotripter nach einem der Ansprüche 6 bis 10, **dadurch gekennzeichnet,** daß die Achse (46) um eine weitere Achse (47) drehbar ist, die senkrecht auf einer Fläche steht, in der die Zentralachse (4) und die Achse (46) liegen.

FIG 1

FIG 2

FIG 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | FR-A-2 591 467 (R. WOLF)<br>* Zusammenfassung; Seite 6, Zeile 33 - Seite 7, Zeile 7; Seite 9, Zeile 16 - Seite 10, Zeile 5; Figur 13 * | 1 | G 10 K 9/12<br>A 61 B 17/22 |
| A | | 2-4,6, 10 | |
| | --- | | |
| Y | US-A-4 382 290 (R.M. HAVIRA)<br>* Zusammenfassung; Spalte 4, Zeile 4 - Spalte 5, Zeile 27 * | 1 | |
| | --- | | |
| A | US-A-4 080 839 (SCHRAIBER et al.)<br>* Insgesamt * | 1 | |
| | --- | | |
| A | EP-A-0 148 653 (DORY)<br>* Zusammenfassung; Seite 3, Zeile 7 - Seite 4, Zeile 34; Figur 1 * | 1,6,7 | |
| | --- | | |
| A | IEEE 1981, ULTRASONICS SYMPOSIUM PROCEEDINGS, Chicago, Illinois, 14.-16. Oktober 1981, Band 2, Seiten 705-710, IEEE, New York, US; J.P. DO-HUU et al.: "Annular array transducer for deep acoustic hyperthermia"<br>* Der ganze Artikel * | 5 | |
| | --- | | **RECHERCHIERTE SACHGEBIETE (Int. Cl.4)** |
| P,A | DE-U-8 628 880 (SIEMENS)<br>* Seite 8, letzter Abschnitt; Figur 3 * | 1-3 | G 10 K<br>G 01 S<br>G 01 N<br>A 61 B |
| | --- | | |
| P,A | FR-A-2 608 913 (TOSHIBA)<br>* Zusammenfassung; Seite 3, Zeile 26 - Seite 5, Zeile 19 * | 1-3 | |
| | ----- | | |

Der vorliegende Recherchenbericht wurde für alle Patentansprüche erstellt

| Recherchenort | Abschlußdatum der Recherche | Prüfer |
|---|---|---|
| DEN HAAG | 12-12-1988 | OLDROYD D.L. |